# EUROPÄISCHE PATENTSCHRIFT

(11) **EP 0 912 524 B1**
(45) Veröffentlichungstag und Bekanntmachung des Hinweises auf die Patenterteilung: **13.03.2002**
(21) Anmeldenummer: 97923045.5
(22) Anmeldetag: 09.05.1997
(51) Int. Cl.: C07D 239/34, C07D 239/52, C07D 239/46, A01N 43/54

(54) **2-(O- PYRIMIDIN-4-YL]METHYLENOXY)PHENYLESSIGSÄURE-DERIVATE UND IHRE VERWENDUNG ZUR BEKÄMPFUNG VON SCHADPILZEN UND TIERISCHEN SCHÄDLINGEN**
2-(O- PYRIMIDIN-4-YL]METHYLENE OXY)PHENYL ACETIC ACID DERIVATIVES AND THEIR USE IN COMBATTING NOXIOUS FUNGI AND ANIMAL PESTS
DERIVES D'ACIDE DE 2-(O- PYRIMIDIN-4-YL]METHYLENOXY)PHENYLACETIQUE ET LEUR UTILISATION POUR LUTTER CONTRE LES CHAMPIGNONS NUISIBLES ET LES PARASITES ANIMAUX

(30) Priorität: 22.05.1996 DE 19620614
(43) Veröffentlichungstag der Anmeldung: 06.05.1999
(73) Patentinhaber: BASF AKTIENGESELLSCHAFT, 67056 Ludwigshafen (DE)
(72) Erfinder: RACK, Michael, D-69123 Heidelberg (DE); RÖHL, Franz, D-67105 Schifferstadt (DE); AMMERMANN, Eberhard, D-64646 Heppenheim (DE); HARRIES, Volker, D-67227 Frankenthal (DE); LORENZ, Gisela, D-67434 Hambach (DE); STRATHMANN, Siegfried, D-67117 Limburgerhof (DE); GROTE, Thomas, D-67105 Schifferstadt (DE); GRAMMENOS, Wassilios, D-67063 Ludwigshafen (DE); KIRSTGEN, Reinhard, D-67434 Neustadt (DE); OBERDORF, Klaus, D-69117 Heidelberg (DE); BAYER, Herbert, D-68159 Mannheim (DE); MÜLLER, Bernd, D-67227 Frankenthal (DE); MÜLLER, Ruth, D-67159 Friedelsheim (DE); SAUTER, Hubert, D-68167 Mannheim (DE)
(74) Vertreter: Fitzner, Ulrich, Dr.
(86) Internationale Anmeldenummer: EP9702391
(87) Internationale Veröffentlichungsnummer: WO9744325

(56) Entgegenhaltungen:
- EP-A- 0 350 691
- EP-A- 0 513 580
- GB-A- 2 226 817

## Beschreibung

Die vorliegende Erfindung betrifft 2-(O-[Pyrimidin-4-yl]methylenoxy)phenylessigsäure-Derivate der allgemeinen Formel I 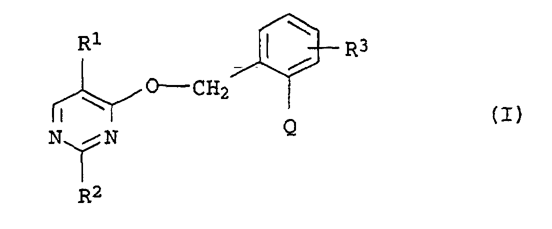 sowie deren Salze und N-Oxide, in der die Reste R¹ bis R³ und Q die folgenden Bedeutungen haben:
- R¹: Halogen, C₁-C₂-Alkyl oder C₁-C₂-Halogenalkyl;
- R²: Wasserstoff; Amino; Hydroxy; Mercapto; Halogen; C₁-C₈-Alkyl, wobei die Alkylreste eine Phenylgruppe tragen können, welche ihrerseits einen oder, unabhängig voneinander, zwei der folgenden Substituenten tragen kann: Halogen, Cyano, Nitro, C₁-C₄-Alkyl, C₁-C₄-Halogenalkyl und C₁-C₄-Alkoxy; C₁-C₈-Halogenalkyl; C₁-C₈-Alkoxy-C₁-C₄-alkyl; C₁-C₈-Alkoxy; C₁-C₈-Monoalkylamino; Di-C₁-C₈-alkylamino; C₁-C₈-Alkylthio; C₁-C₈-Alkylsulfoxyl; C₁-C₈-Alkylsulfonyl; C₃-C₈-Cycloalkyl; Tri-C₁-C₈-alkylsilyloxy oder gegebenenfalls im aromatischen Ring substituiertes: Phenyl, Phenoxy, Phenoxymethyl, Benzyloxy oder Heteroaryl,
wobei R² verschieden ist von R¹;
- R³: Wasserstoff; Cyano; Halogen; C₁-C₄-Alkyl, C₁-C₄-Halogenalkyl oder C₁-C₄-Alkoxy und
- Q: C(=CHCH₃)-COOCH₃ oder C(=CHOCH₃)-COOCH₃.

Daneben betrifft die Erfindung die Verbindungen I enthaltenden Mittel sowie deren Verwendung zur Bekämpfung von Schadpilzen und tierischen Schädlingen, wobei die Behandlung von Tieren ausgenommen bleibt.

[2-Heteroaryloxymethylen)phenyl]-β-methylacrylsäuremethylester mit fungizider und/oder insektizider und akarizider Wirkung sind bereits bekannt (vgl. EP-A 513 580).

Weiterhin sind [2-Heteroaryloxymethylen)phenyl]-β-methoxyacrylsäuremethylester mit fungizider und/oder insektizider und akarizider Wirkung bekannt (vgl. EP-A 178 826, EP-A 278 595, EP-A 350 691, EP-A 407 873).

Die Wirkung der in den vorgenannten Druckschriften beschriebenen Verbindungen gegen Schadpilze und tierische Schädlinge kann noch nicht befriedigen.

Der vorliegenden Erfindung lagen daher neue Verbindungen mit verbesserten Eigenschaften bei der Bekämpfung von Schadpilzen und tierischen Schädlingen als Aufgabe zugrunde.

Demgemäß wurden die eingangs definierten Verbindungen I gefunden sowie sie enthaltende Mittel und ihre Verwendung zur Bekämpfung von Schadpilzen und tierischen Schädlingen.

Die Herstellung der Verbindungen I erfolgt in Analogie zu den in der eingangs genannten Literatur beschriebenen Verfahren.

Bei der Synthese der Verbindungen I ist es im allgemeinen unerheblich, ob zuerst die Gruppe Q oder die 2-(O-[Pyrimidin-4-yl]-methylenoxy)-Gruppe aufgebaut wird.

Man erhält die Verbindungen I beispielsweise dadurch, daß man ein Pyrimidin-4-ol der Formel II in an sich bekannter Weise in einem inerten organischen Lösungsmittel in Gegenwart einer Base mit einem Benzylderivat der Formel III umsetzt.

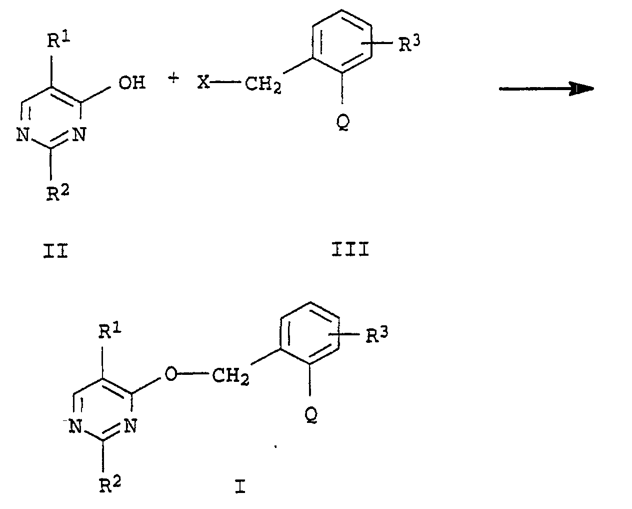

In der Formel III steht X für eine nucleophil austauschbare Abgangsgruppe wie Halogen (z.B. Chlor, Brom oder Iod), Alkylsulfonyl (z.B. Methylsulfonyl oder Trifluormethylsulfonyl) oder Arylsulfonyl (z.B. Phenylsulfonyl oder 4-Methylphenylsulfonyl).

Die Umsetzung wird üblicherweise bei Temperaturen von 0 bis 80, vorzugsweise 20 bis 60 °C durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol, Ketone wie Aceton und Methylethylketon sowie Dimethylsulfoxid, Dimethylformamid, Dimethylacetamid, 1,3-Dimethylimidazolidin-2-on und 1,3-Dimethyltetrahydro-2(1H)-pyrimidinon. Besonders bevorzugt sind Methylenchlorid, Aceton und Dimethylformamid.

Es können auch Gemische der genannten Lösungsmittel verwendet werden.

Als Basen kommen allgemein anorganische Verbindungen mit basischem Charakter wie Alkalimetall- und Erdalkalimetallhydroxide wie Lithiumhydroxid, Natriumhydroxid, Kaliumhydroxid, Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Kaliumcarbonate und Calciumcarbonat sowie Alkalimetallhydrogencarbonate wie Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriummethanolat, Kaliumethanolat, Kalium-tert.-butanolat und Dimethoxymagnesium.

Außerdem eignen sich als Basen organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine.

Besonders bevorzugt sind Natriumhydroxid, Natriumhydrid, Kaliumcarbonat und Kalium-tert.-butanolat.

Die Basen werden im allgemeinen äquimolar oder im Überschuß, gegebenenfalls auch als Lösungsmittel verwendet.

Es kann für die Umsetzung vorteilhaft sein, eine katalytische Menge eines Kronenethers wie z.B. 18-Krone-6 oder 15-Krone-5 zuzusetzen.

Die Umsetzung kann auch in Zweiphasensystemen, bestehend beispielsweise aus einer Lösung von Alkali- oder Erdalkalihydroxiden oder Alkali- oder Erdalkalicarbonaten in Wasser und einer organischen Phase wie z.B. halogenierten Kohlenwasserstoffen, durchgeführt werden. Als Phasentransferkatalysatoren können Ammoniumhalogenide und -tetrafluoroborate wie z.B. Benzyltriethylammoniumchlorid, Benzyltributylammoniumbromid, Tetrabutylammoniumchlorid, Hexadecyltrimethylammoniumbromid oder Tetrabutylammoniumtetrafluoroborat sowie Phosphoniumhalogenide wie Tetrabutylphosphoniumchlorid oder Tetraphenylphosphoniumbromid eingesetzt werden.

Es kann für die Umsetzung vorteilhaft sein, zunächst die Verbindungen II mit Base zu behandeln und das resultierende Salz mit den Verbindungen III umzusetzen.

Die Verbindungen II können durch Kondensation von β-Carbonylverbindungen VI mit Amidinen, Guanidinen, Harnstoffen oder Thioharnstoffen VII in Analogie zu bekannten Verfahren erhalten werden [vgl. J. Chem. Soc. (1946), Seite 5].

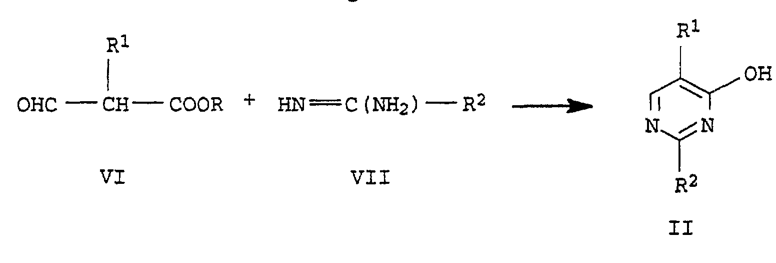

R in der Formel VI steht vor allem für eine C₁-C₄-Alkylgruppe, insbesondere Methyl oder Ethyl.

Die Umsetzung wird üblicherweise bei Temperaturen von 0 bis 120, vorzugsweise 20 bis 80 °C und insbesondere bei der Siedetemperatur des Lösungsmittels durchgeführt. Als Lösungsmittel finden üblicherweise Alkohole, insbesondere Methanol oder Ethanol, Verwendung.

Die Verbindungen VII können auch in Form ihrer Salze, insbesondere als Hydrohalogenide (z.B. Hydrochlorid oder Hydrobromid) eingesetzt werden. Bei der Verwendung von Salzen empfiehlt es sich, die Umsetzung in Gegenwart einer Base durchzuführen (z.B. Erdalkalimetall- oder Alkalimetallalkoholate oder -hydroxide wie Natriummethanolat, Natriumethanolat, Kalium-tert.-butylat, Natriumhydroxid, Kaliumhydroxid und Calciumhydroxid).

Die Ausgangsverbindungen vom Typ II und ihre Synthesen sind allgemein bekannt, und zwar insbesondere aus folgenden Publikationen: 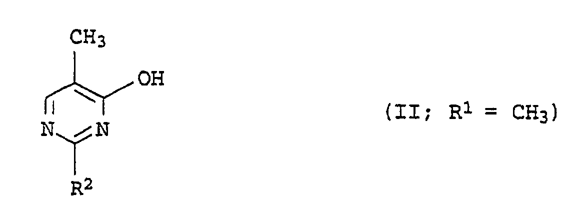 Justus Liebigs Ann. Chem. 758, (1972), Seiten 125, 127, 130;
Chem. Pharm. Bull. 22, (1974), Seiten 1239, 1240, 1245-1247;
J. Amer. Chem. Soc. 79 (1957) Seite 2230;
Bull. Soc. Chim. Fr. (1965), Seiten 2301-2306;
Bull. Soc. Chim. Fr. (1963), Seite 673;
BE-A 645062;
Recl. Trav. Chim. Pays-Bas 87, 10, (1968), Seite 1089;
Chem. Phann. Bull. 36, 5, (1988), Seiten 1669-1675;
Tetrahedron 25, (1969), Seiten 5989, 5992;
J. Org. Chem. 35, (1970), Seiten 3786, 3790, 3791;
Z. Chem., GE 25, 9, (1985), Seiten 328-329;
J. Chem. Soc. (1950), Seiten 452, 456, 458;
Am. Chem. J. 29 (1903),Seite 487;
Bull. Soc. Chim. Belg. 68 (1959), Seiten 30, 40;
J. Biol .Chem. 3 (1907), Seite 303;
Arch. Pharm. (Weinheim Ger.) GE, 317, 5, (1984), Seiten 425-430;
Am. Chem. J. 31 (1904), Seite 595;
Am. Chem. J. 43 (1910), Seite 23;
J. Amer. Chem. Soc. 51 (1929), Seite 1240;
Am. Chem. J. 42 (1909), Seite 368;
Rocz. Chem. 51, (1977), Seiten 1227, 1228, 1230;
Pol. J. Chem. EN, 55, 7/8, (1981), Seiten 1673-1676;
Org. Mass Spectrom. 14, (1979), Seiten 405, 409, 412;
Aust. J. Chem. 41, 8, 1988, 1209-1219;
Pol. J. Chem. 57, 7-9, (1983), Seiten 1027-1031; 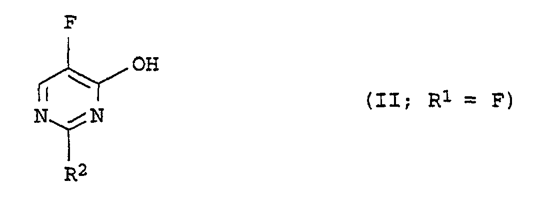 CS-A 107166;
BE-A 627342;
US-A 3954759;
US-A 3954758;
CS-A 108806;
SU-A 232975;
BE-A 860309;
JP-A 50150936;
Gazz. Chim. Ital. 93 (1963) Seiten 1268, 1272;
Collect. Czech. Chem. Commun. 27 (1962) Seiten 2250 -2560;
J. Med. Chem. 8 (1965), Seiten 253;
J. Org. Chem. 27 (1962), Seite 2580;
J. Chem. Soc. C. (1967), Seite 1822;
J. Chem. Soc. C. (1967), Seiten 2206 - 2207;
J. Med. Chem. 36, 18 (1993), Seiten 2627-2638;
J. Chem. Soc. (1959), Seiten 3278, 3284;
J. Am. Chem. Soc. 79 (1957), Seite 4559;
Acta Chem. Scand. 23 (1969), Seite 294. 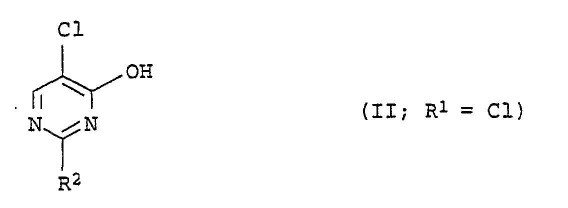 BE-A 645062;
GB-A 1174165;
Collect. Czech. Chem. Commun. 27 (1962), Seiten 2250 - 2560;
J. Org. Chem. 27 (1962), Seiten 3507, 3510;
J. Med. Chem. 6 (1963), Seiten 688-693;
J. Med. Chem. 36, 18 (1993), Seiten 2627-2638. 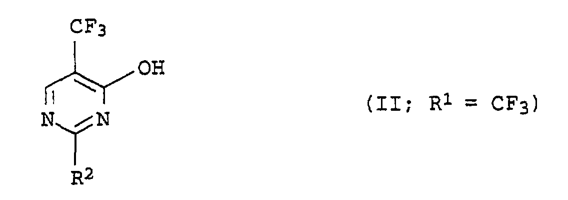 Heterocycles 31, 3 (1990), Seiten 569 - 574.

Die Herstellung der Verbindungen III, in denen Q für C(=CHCH₃)-COOCH₃ steht, ist bekannt aus EP-A 513 580.

Die Herstellung der Verbindungen III, in denen Q für C(=CHOCH₃)-COOCH₃ steht, ist bekannt aus EP-A 203 606.

Die Verbindungen I können bei der Herstellung aufgrund ihrer C=C-Doppelbindungen in der Gruppe Q als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung der Isomeren nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen, in der behandelten Pflanze oder im zu bekämpfenden Schadpilz oder tierischen Schädling erfolgen.

In bezug auf die C=N-Doppelbindung in der Gruppe Q werden hinsichtlich ihrer Wirksamkeit die E-Isomere der Verbindungen I bevorzugt (Konfiguration bezogen auf die OCH₃-Gruppe bzw. die CH₃-Gruppe im Verhältnis zur -COOCH₃-Gruppe.

Teil der Erfindung sind auch die Salze der säurebeständigen Verbindungen I, welche basische Zentren, vor allem basische Stickstoffatome enthalten, insbesondere mit Mineralsäuren wie Schwefelsäure und Phosphorsäure oder Lewis-Säuren wie Zinkchlorid. Üblicherweise kommt es hierbei auf die Art des Salzes nicht an. Im Sinne der Erfindung sind solche Salze bevorzugt, die die von Schadpilzen oder tierischen Schädlingen freizuhaltenden Pflanzen, Flächen, Materialien oder Räume nicht schädigen und die Wirkung der Verbindungen I nicht beeinträchtigen. Besonders bedeutsam sind derartige Salze, welche für landwirtschaftliche Zwecke geeignet sind.

Die Salze der Verbindungen I sind in an sich bekannter Weise zugänglich, vor allem durch Umsetzen der entsprechenden Verbindungen I mit den genannten Säuren in Wasser oder einem inerten organischen Lösungsmittel bei Temperaturen von (-80) bis 120, vorzugsweise 0 bis 60 °C.

Die Verbindungen der Formel I können auch nach bekannten Methoden oder analog zu diesen in ihre N-Oxide übergeführt werden {vgl. z.B. A. Albini u. S. Pietra, Heterocyclic N-Oxides, CRC-Press Inc., Boca Raton, USA 1991; H.S. Mosher et al., Org. Synth. Coll. Vol. IV, 1963, Seite 828; E.C, Taylor et al., Org. Synth. Coll. Vol. IV, 1963, Seite 704; T.W. Bell et. al., Synth. 69, 226 (1990)}.

Unter den zur Oxidation des Pyridinrings üblichen Oxidationsmitteln seien beispielhaft Peressigsäure, Trifluorperessigsäure, Perbenzoesäure, m-Chlorperbenzoesäure, Monomaleinpersäure, Magnesiummonoperoxyphthalat, Natriumperborat, Oxone® (enthält Peroxomonosulfat), Perwolframsäure und Wasserstoffperoxid genannt.

Geeignete Lösungsmittel sind z.B. Wasser, Schwefelsäure, Carbonsäuren wie Essigsäure und Trifluoressigsäure sowie halogenierte Kohlenwasserstoffe wie Dichlormethan und Chloroform.

Normalerweise gelingt die Oxidation bei Temperaturen von 0°C bis Siedetemperatur des Reaktionsgemisches.

Das Oxidationsmittel wird normalerweise in mindestens äquimolaren Mengen, bezogen auf die Ausgangsverbindung, eingesetzt. Im allgemeinen hat sich aber ein großer Überschuß an Oxidationsmittel als besonders vorteilhaft erwiesen.

Bei den eingangs angegebenen Definitionen der Verbindungen I wurden Sammelbegriffe verwendet, die allgemein repräsentativ für die folgenden Gruppen stehen:
Halogen: Fluor, Chlor, Brom und Jod;
Alkyl: geradkettige oder verzweigte Alkylgruppen mit 1 bis 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3 -Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Alkylamino: eine Aminogruppe, welche eine geradkettige oder verzweigte Alkylgruppe mit 1 bis 6 Kohlenstoffatomen wie vorstehend genannt trägt;
Dialkylamino: eine Aminogruppe, welche zwei voneinander unabhängige, .geradkettige oder verzweigte Alkylgruppen mit jeweils 1 bis 6 Kohlenstoffatomen wie vorstehend genannt, trägt;
Alkylsulfoxyl: z.B. C₁-C₈-Alkylsulfoxyl: Alkylgruppen, wie vorstehend definiert, welche über eine Sulfoxylgruppe (-SO₂-) an das Gerüst gebunden sind;
Alkylsulfonyl: z.B. C₁-C₈-Alkylsulfoxyl: Alkylgruppen, wie vorstehend definiert, welche über eine Sulfoylgruppe (-SO-) an das Gerüst gebunden sind;
Trialkylsilyloxy: z.B. Tri-C₁-C₈-alkylsilyloxy:
Silyloxygruppen 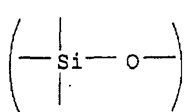 welche am Siliciumatom drei
Alkylgruppen, wie vorstehend definiert, tragen und über das Sauerstoffatom an das Gerüst gebunden sind;
Halogenalkyl: geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit 1 bis 4, 6 oder 8 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
Alkoxyalkyl: z.B. C₁-C₈-Alkoxy-C₁-C₄-alkyl: Alkylgruppen mit 1 bis 4 Kohlenstoffatomen, wie vorstehend definiert, welche eine Alkoxygruppe mit 1 bis 8 Kohlenstoffatomen, wie vorstehend genannt, tragen;
Alkylendioxy: z.B. C₁-C₂-Alkylendioxy: geradkettige oder verzweigte Alkylengruppen mit 1 bis 2 Kohlenstoffatomen, welche an zwei Stellen über je ein Sauerstoffatom (-O-) in das Gerüst eingebunden oder an das Gerüst gebunden sind wie Methylendioxy (-O-CH₂-O-) oder Ethylendioxy (-O-CH₂CH₂-O-);
Alkylthio: geradkettige oder verzweigte Alkylgruppen mit 1 bis 4 oder 6 Kohlenstoffatomen wie vorstehend genannt, welche über ein Schwefelatom (-S-) an das Gerüst gebunden sind, z.B. C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
Cycloalkyl: z.B. C₃-C₈-Cycloalkyl: monocyclische Alkylgruppen mit 3 bis 8 Kohlenstoffringgliedern, z.B. Cyclopropyl, Cyclobutyl, Cyclopentyl und Cyclohexyl;
Heteroaryl: aromatische mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können, z.B.

- 5-gliedriges Heteroaryl, enthaltend ein bis drei Stickstoffatome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
- 5-gliedriges Heteroaryl, enthaltend ein bis vier Stickstoffatome oder ein bis drei Stickstoffatome und ein Schwefel- oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
- benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
- über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien- 1,4-diylgruppe verbrückt sein können, wobei diese Ringe über eines der Stickstoffringglieder an das Gerüst gebunden sind;
- 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
- benzokondensiertes 6-gliedriges Heteroaryl, enthaltend ein bis vier Stickstoffatome; 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome, wie vorstehend genannt, ersetzt sein können.

Die Angabe "gegebenenfalls substituiert" soll zum Ausdruck bringen, daß in den derart charakterisierten Gruppen die Wasserstoffatome zum Teil oder vollständig durch gleiche oder verschiedene beispielsweise derjenigen Gruppen ersetzt sein können, die unter den vorstehend ausgeführten Sammelbegriffen genannt sind.

Im Hinblick auf ihre biologische Wirkung gegen Schadpilze und tierische Schädlinge sind Verbindungen I bevorzugt, in denen die Reste in den folgenden Bedeutungen, und zwar für sich allein oder in Kombination, stehen;
- R¹: Halogen, insbesondere Fluor und Chlor;
- R¹: Methyl;
- R¹: Trifluormethyl;
- R²: C₁-C₈-Alkyl, wobei dieser Rest partiell oder vollständig halogeniert sein kann;
- R²: C₃-C₆-Cycloalkyl;
- R²: C₁-C₈-Alkoxy;
- R²: C₁-C₈-Alkylthio;
- R²: Di-C₁-C₈-alkylamino;
- R²: C₁-C₈-Alkylsulfoxyl;
- R²: C₁-C₈-Alkylsulfonyl;
- R²: Chlor;
- R²: Phenyl, wobei dieser Rest eine bis drei der folgenden Gruppen tragen kann: Halogen, C₁-C₄-Alkyl, Cyano, C₁-C₄-Alkoxy, Nitro, gegebenenfalls teilweise oder vollständig halogeniertes C₁-C₂-Alkylendioxy;
- R²: durch Phenyl substituiertes C₁-C₄-Alkyl, wobei der Alkylteil ansonsten unsubstituiert ist und der Phenylteil eine bis zwei der folgenden Gruppen tragen kann: Halogen, Cyano, Nitro, C₁-C₄-Alkyl, C₁-C₄-Halogenalkyl, C₁-C₄-Alkoxy;
- R³: Wasserstoff.

Im Hinblick auf die genannte biologische Wirkung besonders bevorzugt sind die in den folgenden Tabellen zusammengestellten Verbindungen I.

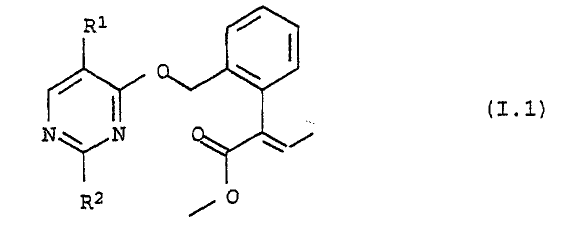

**Tabelle 1**

| |
|---|
| Verbindungen der Formel I.1, mit |
| R¹ = Methyl; |
| R² = jeweils eine Zeile der Tabelle A |

**Tabelle 2**

| |
|---|
| Verbindungen der Formel I.1, mit |
| R¹ = Fluor; |
| R² = jeweils eine Zeile der Tabelle A |

**Tabelle 3**

| |
|---|
| Verbindungen der Formel I.1, mit |
| R¹ = Chlor; |
| R² = jeweils eine Zeile der Tabelle A |

**Tabelle 4**

| |
|---|
| Verbindungen der Formel I.1, mit |
| R¹ = Trifluormethyl; |
| R² = jeweils eine Zeile der Tabelle A |

**Tabelle 5**

| |
|---|
| Verbindungen der Formel I.1, mit |
| R¹ = Chlor; |
| R² = jeweils eine Zeile der Tabelle A |

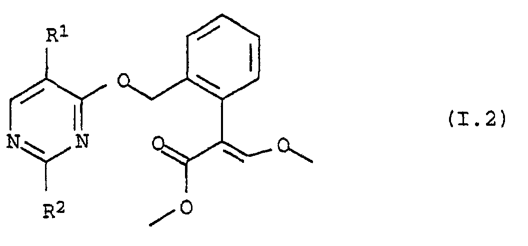

**Tabelle 6**

| |
|---|
| Verbindungen der Formel I.2, mit |
| R¹ = Methyl; |
| R² = jeweils eine Zeile der Tabelle A |

**Tabelle 7**

| |
|---|
| Verbindungen der Formel I.2, mit |
| R¹ = Fluor; |
| R² = jeweils eine Zeile der Tabelle A |

**Tabelle 8**

| |
|---|
| Verbindungen der Formel I.2, mit |
| R¹ = Chlor; |
| R² = jeweils eine Zeile der Tabelle A |

**Tabelle 9**

| |
|---|
| Verbindungen der Formel I.2, mit |
| R¹ = Trifluormethyl; |
| R² = jeweils eine Zeile der Tabelle A |

**Tabelle 10**

| |
|---|
| Verbindungen der Formel I.2, mit |
| R¹ = Chlor; |
| R² = jeweils eine Zeile der Tabelle A |

**Tabelle A**

| Nr. | R² |
|---|---|
| 1 | H |
| 2 | Methyl |
| 3 | Ethyl |
| 4 | n-Propyl |
| 5 | i-Propyl |
| 6 | Cyclopropyl |
| 7 | n-Butyl |
| 8 | s-Butyl |
| 9 | i-Butyl |
| 10 | t-Butyl |
| 11 | Cyclobutyl |
| 12 | n-Pentyl |
| 13 | i-Pentyl |
| 14 | neo-Pentyl |
| 15 | Cyclopentyl |
| 16 | n-Hexyl |
| 17 | n-Heptyl |
| 18 | Trifluormethyl |
| 19 | Chlormethy |
| 20 | O-Methyl |
| 21 | O-Ethyl |
| 22 | O-n-Propyl |
| 23 | O-i-Propyl |
| 24 | O-n-Butyl |
| 25 | O-s-Butyl |
| 26 | O-i-Butyl |
| 27 | O-t-Butyl |
| 28 | O-n-Pentyl |
| 29 | O-i-Pentyl |
| 30 | O-neo-Pentyl |
| 31 | O-n-Hexyl |
| 32 | S-Methyl |
| 33 | S-Ethyl |
| 34 | S-n-Propyl |
| 35 | S-i-Propyl |
| 36 | S-n-Butyl |
| 37 | S(=O)-Methyl |
| 38 | S(=O)-Ethyl |
| 39 | S(=O)-n-Propyl |
| 40 | S(=O)-i-Propyl |
| 41 | S(=O)-n-Butyl |
| 42 | S(=O)₂-Methyl |
| 43 | S(=O)₂-Ethyl |
| 44 | S(=O)₂-n-Propyl |
| 45 | S(=O)₂-i-Propyl |
| 46 | S(=O)₂-n-Butyl |
| 47 | NH₂ |
| 48 | N(Methyl)₂ |
| 49 | N(Ethyl)₂ |
| 50 | N(n-Propyl)₂ |
| 51 | Chlor |
| 52 | O-Phenyl |
| 53 | O-CH₂-Phenyl |
| 54 | O-CH₂-(4-Chlorphenyl) |
| 55 | Phenyl |
| 56 | 2-F-Phenyl |
| 57 | 3-F-Phenyl |
| 58 | 4-F-Phenyl |
| 59 | 2-Cl-Phenyl |
| 60 | 3-ClPhenyl |
| 61 | 4-Cl-Phenyl |
| 62 | 2-Br-Phenyl |
| 63 | 3-Br-Phenyl |
| 64 | 4-Br-Phenyl |
| 65 | 2-Methyl-Phenyl |
| 66 | 3-Methyl-Phenyl |
| 67 | 4-Methyl-Phenyl |
| 68 | 2-CN-Phenyl |
| 69 | 3-CN-Phenyl |
| 70 | 4-CN-Phenyl |
| 71 | 2-OMe-Phenyl |
| 72 | 3-OMe-Phenyl |
| 73 | 4-OMe-Phenyl |
| 74 | 2-CN-Phenyl |
| 75 | 3-CN-Phenyl |
| 76 | 4-CN-Phenyl |
| 77 | 2-Nitro-Phenyl |
| 78 | 3-Nitro-Phenyl |
| 79 | 4-Nitro-Phenyl |
| 80 | 2,4-Cl₂-Phenyl |
| 81 | 2,4-(CH₃)₂-Phenyl |
| 82 | 3,4-Cl₂-Phenyl |
| 83 | 3,4-Methylendioxy-Phenyl |
| 84 | 3,4-(Difluormethylendioxy)-Phenyl |
| 85 | 2,4-F₂-Phenyl |
| 86 | CH₂-Phenyl |
| 87 | CH₂-(3-Cl-Phenyl) |
| 88 | CH₂-(4-Cl-Phenyl) |
| 89 | 2-CF₃-Phenyl |
| 90 | 3-CF₃-Phenyl |
| 91 | 4-CF₃-Phenyl |

Unter den vorstehend zusammengestellten besonders bevorzugten Verbindungen I sind diejenigen ganz besonders bevorzugt, in denen R² nicht Wasserstoff bedeutet.

Die Verbindungen I eignen sich zur Bekämpfung von Schadpilzen und tierischen Schädlingen.

Sie können in Abhängigkeit von ihren chemischen und physikalischen Eigenschaften mit üblichen, also dem Fachmann geläufigen, Formulierungshilfsmitteln formuliert werden. Die Produkte dieses Vorgangs werden als "Mittel" bezeichnet.

Geeignete Formulierungshilfsmittel sind z.B. feste oder flüssige Trägerstoffe, oberflächenaktive Mittel und Haftmittel.

Unter flüssigen Trägerstoffen werden flüssige Lösungsmittel wie Wasser und organische Lösungsmittel verstanden, wobei letztere vor allem bei Verwendung von Wasser als Lösungsmittel die Funktion eines Hilfslösungsmittels haben. Als organische Lösungsmittel können verwendet werden: Aromaten wie Xylol, Toluol und Alkylnaphthaline, chlorierte Aromaten oder chlorierte aliphatische Kohlenwasserstoffe wie Chlorbenzole, Chlorethylene und Methylenchlorid, aliphatische Kohlenwasserstoffe wie Cyclohexan und Paraffine, z.B. Mineralölfraktionen, Alkohole wie Butanol, iso-Butanol, Cyclohexanol und Glykol sowie die zugehörigen Ether und Ester, Ketone wie Aceton, Methylethylketon, Methyliso-butylketon und Cyclohexanon, aprotisch dipolare Lösungsmittel wie Dimethylformamid, N-Methyl-2-pyrrolidon und Dimethylsulfoxid.

Als feste Trägerstoffe kommen beispielsweise in Betracht: Natürliche Gesteinsmehle und Mineralerden wie Kieselsäuren, Silicate, Kaoline, Tonerden, Bolus, Löß, Talkum, Kreide, Kalkstein, Kalk, Dolomit, Magnesiumoxid, Quarz, Attapulgit, Montmorillonit und Diatomeenerde; synthetische Gesteinsmehle wie hochdisperse Kieselsäure oder Mehle von synthetischem Aluminiumoxid und von synthetischen Silikaten. Insbesondere für Granulate geeignete feste Trägerstoffe sind beispielsweise: Gebrochene und fraktionierte natürliche Gesteine wie Calcit, Marmor, Bims, Sepiolith; synthetische Granulate aus anorganischen und organischen Mehlen; Granulate aus organischem Material wie Sägemehl, Kokosnußschalen, Maiskolben oder Tabakstengel.

Geeignete oberflächenaktive Mittel sind nichtionogene und anionische Emulgiermittel/schaumerzeugende Mittel und Dispergiermittel:
- Fettsäure-Polyoxyethylenester wie Laurylalkohol-Polyoxyethylenetheracetat,
- Alkyl-Polyoxyethylen- oder -Polyoxypropylenether etwa von iso-Tridecylalkohol und Fettalkohol-Polyoxyethylenether,
- Alkylarylalkohol-Polyoxyethylenether wie Octylphenol-Polyoxyethylenether,
- Tributylphenol-Polyoxyethylenether,
- ethoxyliertes iso-Octyl-, Octyl- oder Nonylphenol oder Rizinusöl,
- Sorbitester,
- Arylsulfonsäuren, Alkylsulfonsäuren, Alkylschwefelsäuren,
- Alkali-, Erdalkali- und Ammoniumsalze von Arylsulfonsäuren, z.B. Lignin-, Phenol-, Naphthalin- und Dibutylnaphthalinsulfonsäure, Alkylsulfonsäuren, Alkylarylsulfonsäuren, Alkyl-, Laurylether- und Fettalkoholschwefelsäuren, Fettsäuren, sulfatierten Hexa-, Hepta- und Octadecanolen und Fettalkoholglykolethern,
- Kondensationsprodukte von sulfoniertem Naphthalin und seiner Derivate mit Formaldehyd,
- Kondensationsprodukte von Naphthalinsulfonsäuren mit Phenol und Formaldehyd,
- Eiweißhydrolysate und
- insbesondere als Dispergiermittel: Lignin-Sulfitablaugen und Methylcellulose.

Als Haftmittel eignen sich beispielsweise: Carboxymethylcellulose; natürliche und synthetische pulverige, körnige oder latexförmige Polymere wie Gummiarabicum, Polyvinylalkohol, Polyvinylacetat, natürliche Phospholipide wie Kephaline und Lecithine, synthetische Phospholipide.

Weiterhin können die Mittel einen oder mehrere Vertreter der folgenden Stoffgruppen enthalten: Farbstoffe, andere bekannte Wirkstoffe, Spurennährstoffe und weitere Additive.

Als Farbstoffe kommen z.B. anorganische Pigmente wie Eisenoxid, Titanoxid, Ferrocyanblau, ferner organische Pigmente wie Alizarin-, Azo- und Metallphthalocyaninfarbstoffe in Betracht. Unter anderen bekannten Wirkstoffen sind etwa andere Fungizide sowie Insektizide, Akarizide, Herbizide und Wachstumsregulatoren zu verstehen. Spurennährstoffe sind beispielsweise Salze von Eisen, Mangan, Bor, Kupfer, Kobalt, Molybdän und Zink. Als weitere Additive sind etwa mineralische und vegetabile Öle geeignet.

Die Mittel können darüberhinaus mit sonstigen, praktisch bedeutsamen Mischungspartnern wie Düngemittel oder sonstige fertige wirkstoffhaltige Mittel vermischt sein.

Die Herstellung der Mittel erfolgt in an sich bekannter Weise, nämlich in Abhängigkeit von den chemischen und physikalischen Eigenschaften der eingesetzten Stoffe z.B. durch Mischen, gemeinsames Vermahlen, Aufsprühen, Extrudieren, Granulieren oder Auflösen in Wasser, letzteres ggf. unter Zuhilfenahme eines organischen Lösungsmittels. Pulver, Streu- und Stäubemittel sind z.B. durch Mischen oder gemeinsames Vermahlen der Verbindungen I mit einem festen Trägerstoff erhältlich.

Bei den Mitteln handelt es sich in Abhängigkeit von den eingesetzten Stoffen z.B. um Lösungen, Emulsionen, Suspensionen, Pulver, Schäume, Pasten, Granulate, Aerosole oder Feinstverkapselungen in polymeren Stoffen oder in Saatgut-Hüllmassen.

Zur Anwendung werden die für den Handel in der Regel als Konzentrate vorliegenden Mittel gegebenenfalls wie üblich aufgelöst, verdünnt usw., bei Spritzpulvern, wasserdispergierbaren Granulaten, emulgierbaren Konzentraten, Dispersionen und teilweise auch bei Mikrogranulaten normalerweise unter Verwendung von Wasser. Staubförmige und granulierte Zubereitungen sowie versprühbare Lösungen werden vor der Anwendung meist nicht mehr mit weiteren inerten Stoffen verdünnt.

Die Ausbringung der Mittel erfolgt in an sich bekannter Weise, etwa durch Versprühen, Vernebeln, Verstäuben, Verstreuen oder Gießen. Die Pflanzen werden in der Regel mit den Mitteln besprüht oder bestäubt. Alternativ oder zusätzlich behandelt man die Samen der Pflanzen in an sich bekannter Weise.

Beispiele für solche Zubereitungen sind:
I. eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
II. eine Mischung aus 20 Gew.-Teilen einer erfindungsgemäßen Verbindung I, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen des Calciumsalzes der Dodecylbenzolsulfonsäure, 5 Gew.-Teilen des Anlagerungsproduktes von 40 Mol Ethylenoxid an 1 Mol Ricinusöl: durch feines Verteilen der Lösung in Wasser erhält man eine Dispersion;
III. eine wäßrige Dispersion aus 20 Gew.-Teilen einer erfindungsgemäßen Verbindung I, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen einer erfindungsgemäßen Verbindung I, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen einer erfindungsgemäßen Verbindung I, 3 Gew.-Teilen des Natriumsalzes der Diisobutylnaphtalin-1-sulfonsäure, 10 Gew.-Teilen des Natriumsalzes einer Ligninsulfonsäure aus einer Sulfitablauge und 7 Gew.-Teilen pulverförmigem Kieselsäuregel: durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 68 Gew.-Teilen eines paraffinischen Mineralöls.

Werden die Verbindungen I als solche appliziert, so kommt es vor allem auf deren feine Verteilung an.

Die Verbindungen I und die erfindungsgemäßen Mittel zeichnen sich durch eine hervorragende Wirksamkeit gegen ein breites Spektrum von Schadpilzen (pflanzenpathogene Pilze), insbesondere aus der Klasse der
- Ascomyceten,
- Basidiomyceten,
- Deuteromyceten und
- Phycomyceten
aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen sowie an den Samen dieser Pflanzen.

Die Verbindungen I, ihre Salze und N-Oxide sowie die erfindungsgemäßen Mittel werden angewendet, indem man die Schadpilze, deren Lebensraum oder die vor Pilzbefall zu schützenden Saatgüter, Pflanzen, Flächen, Materialien oder Räume mit einer fungizid wirksamen Menge der Mittel oder der Verbindungen I behandelt. Die Anwendung kann vor oder nach dem Befall durch die Pilze erfolgen.

Speziell eignen sich die erfindungsgemäßen Mittel und die Verbindungen I zur Bekämpfung folgender Pflanzenkrankheiten:

Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Pseudoperonospora-Arten in Hopfen und Gurken, Alternaria-Arten an Gemüse und Obst.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)disulfid;
Nitroderivate wie Dinitro-(1-methylheptyl)phenylcrotonat, 2-sec.-Butyl-4,6-dinitrophenyl-3,3-dimethylacrylat, 2-sec.-Butyl-4,6-di-nitrophenyl-iso-propylcarbonat, 5-Nitro-iso-phthalsäure-di-iso-propylester;
heterocyclische Substanzen wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)phosphinyl]-3-phenyl-1,2,4-triazol, 2,3-Dicyano-1,4-dithioanthrachinon, 2-Thio-1,3-dithiolo-[4,5-b]-chinoxalin, 1-(Butylcarbamoyl)-2-benzimidazol-carbaminsäuremethylester, 2-Methoxycarbonylamino-benzimidazol, 2-(Furyl-(2))benzimidazol, 2-(Thiazolyl-(4))benzimidazol, N-(1,1,2,2-Tetrachlorethylthio)tetrahydrophthalimid, N-Trichlormethylthio-tetrahydrophthalimid, N-Trichlor-methylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlor-ethylacetal, Piperazin-1,4-diylbis-(1-(2,2,2-trichlor-ethyl)formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecylmorpholin bzw. dessen Salze, N-[3-(p-tert.-Butylphenyl)-2-methylpropyl]-cis-2,6-dimethylmorpholin, N-[3-(p-tert.-Butylphenyl) -2-methylpropyl] -piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlor-phenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)benzol, 1,2-Bis-3-methoxycarbonyl-2-thioureido)benzol, [2-(4-Chlorphenyl)ethyl]-(1,1-dimethylethyl)-1H-1,2,4-triazol-1-ethanol sowie verschiedene Fungizide wie Dodecylguanidinacetat, 3-[3-(3,5-Dimethyl-2-oxycyclohexyl)-2-hydroxyethyl)]glutarimid, Hexachlorbenzol, DL-Methyl-N-(2,6-dimethyl-phenyl)-N-furoyl (2) alaninat, DL-M-(2,6-Dimethyl-phenyl)-N-(2'-methoxyacetyl)alanin-methylester, N-(2,6-Dimethylphenyl)-N-chloracetyl-D,L-2-aminobutyrolacton, DL-N-(2,6-Dimethylphenyl)-N-(phenylacetyl)alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorhenyl)-1-iso-propylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)pentyl]-1H-1,2,4-triazol, 2,4-Di-fluor-a-(1H-1,2,4-triazolyl-1-methyl)benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)methylsilyl)methyl)-1H-1,2,4-triazol.

Strobilurine wie Methyl-E-methoximino-[a-(o-tolyloxy)-o-tolyl]acetat, Methyl-E-2-{2-[6-(2-cyanophenoxy)pyrimidin-4-yloxy]-phenyl}-3-methoxyacrylat, Methyl-E-methoximino-[a-(2-phenoxyphenyl)]acetamid, Methyl-E-methoximino- [a-(2,5-dimethylphenoxy)-o-tolyl] acetamid.

Anilinopyrimidine wie N-(4,6-Dimethylpyrimidin-2-yl)anilin, N- [4-Methyl-6- (1-propinyl)pyrimidin-2-yl]anilin, N- (4-Methyl-6-cyclopropyl-pyrimidin-2-yl)anilin.

Phenylpyrrole wie 4-(2,2-difluor-1,3-benzodioxol-4-yl)pyrrol-3-carbonitril.

Zimtsäureamide wie 3-(4-Chlorphenyl)-3-(3,4-dimethoxyphenyl)acrylsäuremorpholid.

(2RS,3SR)-1-[3-(2-Chlorphenyl)-2-[4-fluorphenyl]oxiran-2-ylmethyl]-1H-1,2,4-triazol.

Die Verbindungen der Formel I sind außerdem geeignet, tierische Schädlinge, vor allem aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendicuiatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

### Synthesebeispiele

Die in den nachstehenden Synthesebeispielen wiedergegebenen Vorschriften können unter Abwandlung der Ausgangsverbindungen zur Gewinnung weiterer Vertreter der Verbindungen I benutzt werden. Die physikalischen Daten der demgemäß hergestellten Produkte sind in den jeweils anschließenden Tabellen wiedergegeben.

### I) Herstellung von Verbindungen I, in denen Q für C(=CHCH₃)-COOCH₃ steht

### E-α-[(2-[2-Ethyl-5-methylpyrimidin-4-yl)]oxymethyl)phenyl]-β-methylacrylsäuremethylester (Nr. 1 in Tabelle S1)

0,7 g 2-Ethyl-5-methyl-4-hydroxypyrimidin wurden in 20 ml Dimethylformamid gelöst. Hierzu gab man 1 g fein gepulvertes Kaliumcarbonat und 1,35 g α-(2-Brom-methylphenyl)-β-methylacrylsäuremethylester. Man rührte zwei Stunden bei 50 °C, dann über Nacht bei Raumtemperatur und engte ein. Der Rückstand wurde in Essigsäureethylester aufgenommen und mit wenig Wasser gewaschen. Dann wurden die vereinigten organischen Phasen über Natriumsulfat getrocknet. Nach Abziehen des Lösungsmittels wurde der verbleibende Rückstand an Kieselgel mit Cyclohexan/Essigester 1:1 chromatographiert. Man erhielt 1,1 g der Titelverbindung als farblosen Feststoff.
Fp.: 54 - 55°C

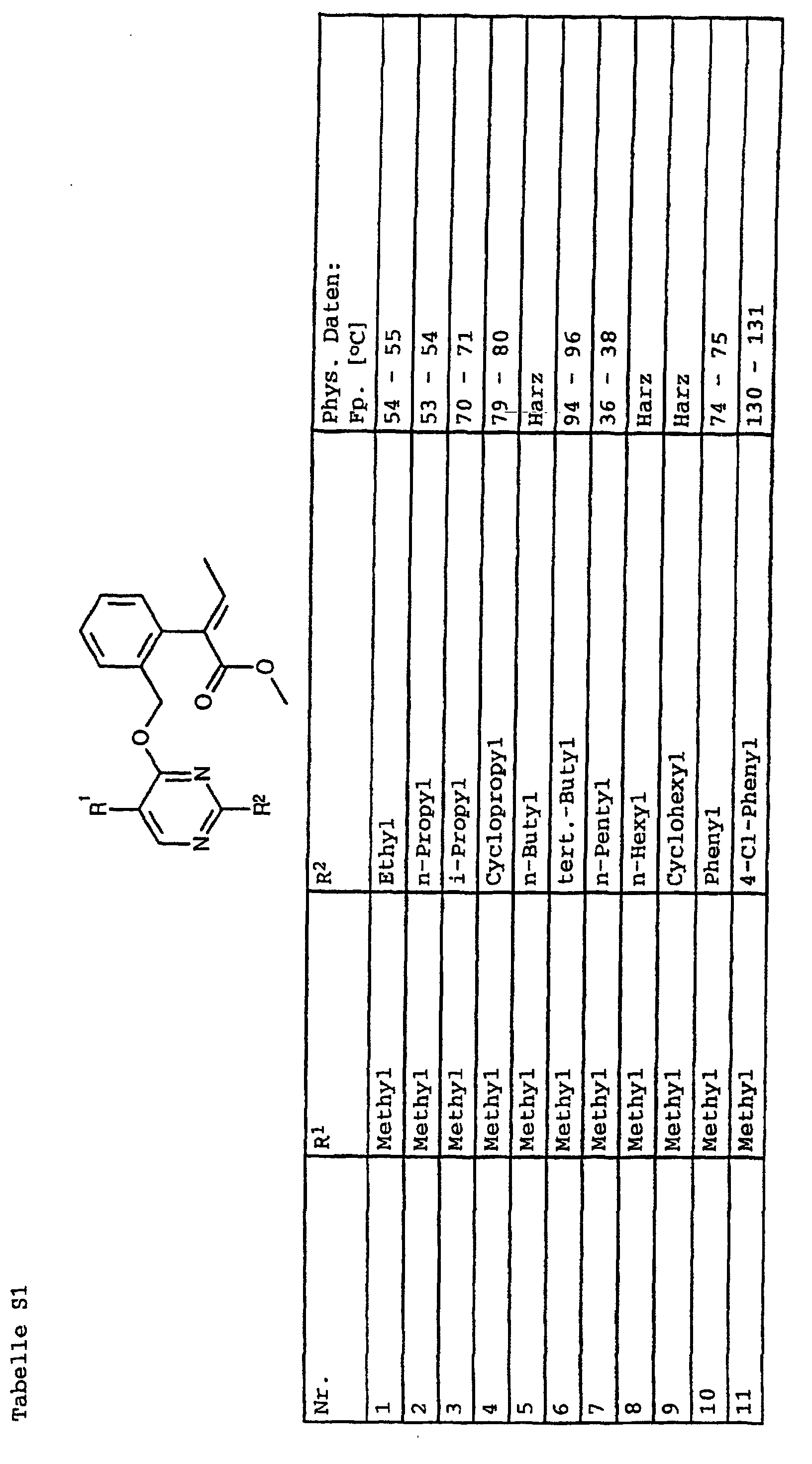

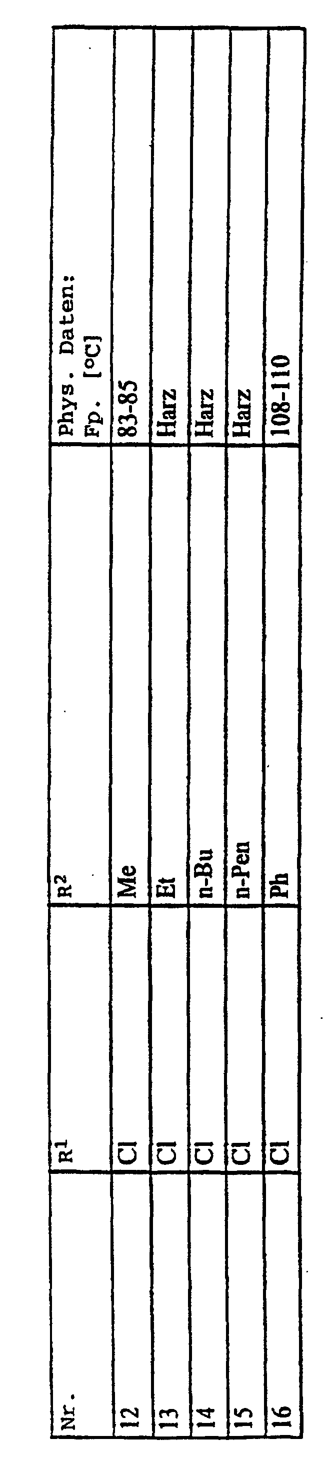

### II) Herstellung von Verbindungen I, in denen Q für C(=CHOCH₃)-COOCH₃ steht

### E-α-[(2- [2-Ethyl-5-methylpyrimidin-4-yl)]oxymethyl)phenyl]-β-methoxyacrylsäuremethylester (Nr. 1 in Tabelle S2)

1,1 g 2-Ethyl-5-methyl-4-hydroxypyrimidin wurden in 25 ml Dimethylformamid gelöst. Hierzu gab man bei 0 °C 0,53 g fein gepulvertes Kaliumhydroxyd. Nach 15 Minuten Rühren bei 0 °C und weiteren 15 Minuten bei Raumtemperatur wurde eine Lösung von 2,3 g α-(2-Brommethylphenyl)-β-methoxyacrylsäuremethylester in 15 ml Dimethylformamid zugetropft. Man rührte 12 Stunden bei Raumtemperatur und engte dann ein. Der Rückstand wurde in Essigester aufgenommen und mit Wasser gewaschen. Dann wurden die vereinigten organischen Phasen über Natriumsulfat getrocknet. Nach Abziehen des Lösungsmittels wurde der verbleibende Rückstand an Kieselgel mit Cyclohexan/Essigester 1:2 chromatographiert. Man erhielt 0,6 g der Titelverbindung als farblosen Feststoff.
Fp.: 76 - 78°C

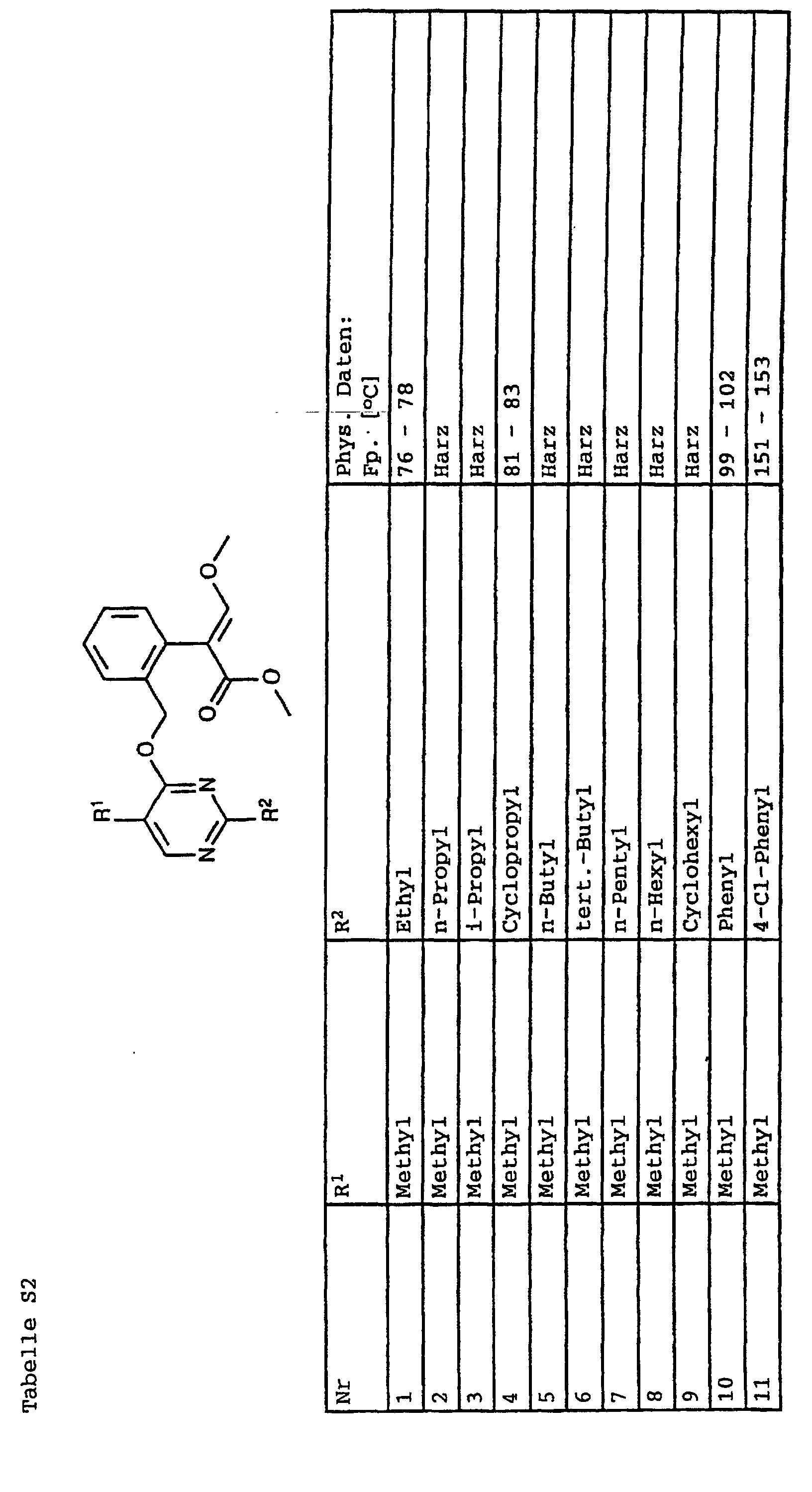

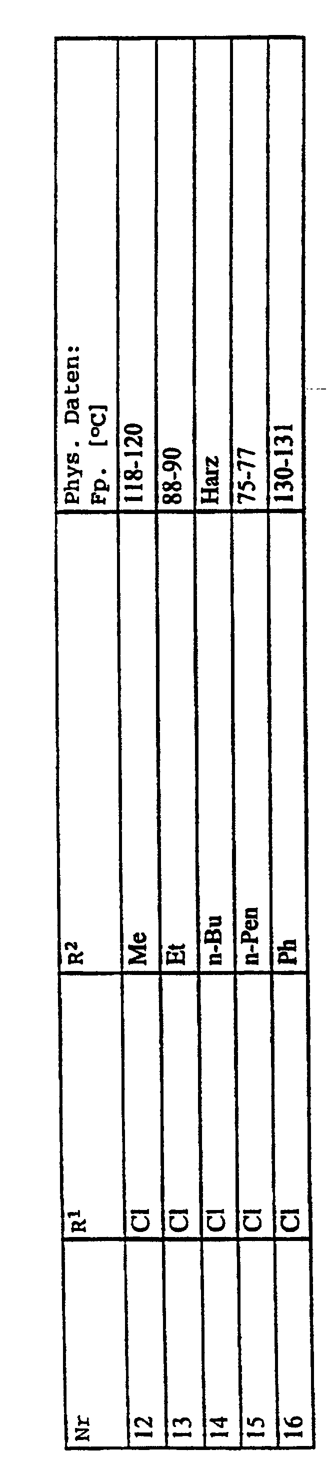

### Anwendungsbeispiele

### 1. Beispiel zur Wirkung gegen Schadpilze

Für die folgenden Versuche zur fungiziden Wirkung der Verbindungen I wurde eine Emulsion verwendet, welche zu 10 Gew.-% aus dem Wirkstoff und zu 90 Gew.-% aus einem Gemisch aus

| | |
|---|---|
| 70 Gew.-% | Cyclohexanol, |
| 20 Gew.-% | Nekanil® LN (Lutensol® AP6, Netzmittel mit Emulgier- und Dispergierwirkung auf der Basis ethoxylierter Alkylphenole) und |
| 10 Gew.-% | Uniperol® EL (nicht-ionischer Emulgator auf Basis von ethoxyliertem Ricinusöl) |

bestand. Die gewünschten Wirkstoff-Konzentrationen wurden durch Verdünnen dieser Emulsion mit Wasser eingestellt. Das Ausmaß des Krankheitsbefalls wurde visuell ermittelt.

### 2. Beispiel zur Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zw. 20 und 22°C und 75 bis 80 % relativer Luftfeuchtigkeit aufgestellt. Nach 7 Tagen wurde das Ausmaß der Mehltauentwicklung ermittelt.

| Wirkstoff | %-Befall der Blätter nach Applikation von 250 ppm-haltiger wäßriger Wirkstoffaufbereitung |
|---|---|
| S1. 2 | 5 |
| S1. 4 | 15 |
| S1. 5 | 5 |
| S1. 7 | 0 |
| S1. 8 | 5 |
| S1. 9 | 15 |
| S1.10 | 15 |
| S2. 1 | 15 |
| S2. 2 | 15 |
| S2. 3 | 15 |
| S2. 4 | 15 |
| S2. 5 | 15 |
| S2. 6 | 15 |
| S2. 7 | 5 |
| S2. 8 | 5 |
| S2 9 | 5 |
| S2.10 | 15 |
| Unbehandelt | 70 |

### 3. Beispiel zur Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 bis 22°C und 65 bis 70 % relativer Luftfeuchte aufgestellt. Nach 8 Tagen wurde das Ausmaß der Rostpilzentwicklung auf den Blättern ermittelt.

| Wirkstoff | %-Befall der Blätter nach Applikation von 250 ppm-haltiger wäßriger Wirkstoffaufbereitung |
|---|---|
| S1. 1 | 0 |
| S1. 2 | 0 |
| S1. 3 | 0 |
| S1. 4 | 5 |
| S1. 5 | 0 |
| S1. 7 | 0 |
| S1. 8 | 0 |
| S1. 9 | 0 |
| S2. 1 | 0 |
| S2. 2 | 0 |
| S2. 3 | 0 |
| S2. 4 | 0 |
| S2. 5 | 0 |
| S2. 6 | 5 |
| S2. 7 | 0 |
| S2. 8 | 0 |
| S2. 9 | 0 |
| Unbehandelt | 80 |

### 4. Beispiele zur Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Mekanil® LN (Lutensol® AP6, Netzmittel mit Emulgier- und Dispergierwirkung auf der Basis ethoxylierter Alkylphenole) und 10 Gew.-% Uniperol® EL (nicht-ionischer Emulgator auf Basis von ethoxyliertem Ricinusöl)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. 2-(O-[Pyrimidin-4-yl]methylenoxy)phenylessigsäure-Derivate der allgemeinen Formel I 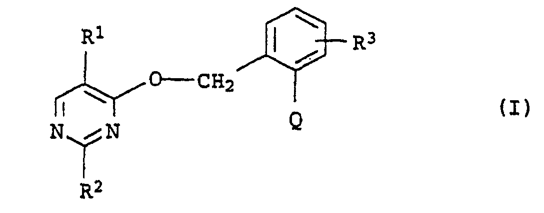 sowie deren Salze und N-Öxide, in der die Reste R¹ bis R³ und Q die folgenden Bedeutungen haben:
R¹ Halogen, C₁-C₂-Alkyl oder C₁-C₂-Halogenalkyl;
R2 Wasserstoff; Amino; Hydroxy; Mercapto; Halogen; C₁-C₈-Alkyl, wobei die Alkylreste eine Phenylgruppe tragen können, welche ihrerseits einen oder, unabhängig voneinander, zwei der folgenden Substituenten tragen kann: Halogen, Cyano, Nitro, C₁-C₄-Alkyl, C₁-C₄-Halogenalkyl und C₁-C₄-Alkoxy; C₁-C₈-Halogenalkyl; C₁-C₈-Alkoxy-C₁-C₄-alkyl; C₁-C₈-Alkoxy; C₁-C₈-Monoalkylamino; Di-C₁-C₈-alkylamino; C₁-C₈-Alkylthio; C₁-C₈-Alkylsulfoxyl; C₁-C₈-Alkylsulfonyl; C₃-C₈-Cycloalkyl; Tri-C₁-C₈-alkylsilyloxy oder gegebenenfalls im aromatischen Ring substituiertes: Phenyl, Phenoxy, Phenoxymethyl, Benzyloxy oder Heteroaryl,
wobei R² verschieden ist von R¹;
R3 Wasserstoff; Cyano; Halogen; C₁-C₄-Alkyl; C₁-C₄-Halogenalkyl oder C₁-C₄-Alkoxy und
Q C(=CHCH₃)-COOCH₃ oder
C(=CHOCH₃)-COOCH₃.

2. Zur Bekämpfung von Schadpilzen und tierischen Schädlingen geeignete Mittel, enthaltend eine wirksame Menge einer Verbindung der allgemeinen Formel I oder eines ihrer Salze oder N-Oxide gemäß Anspruch 1 und mindestens ein Formulierungshilfsmittel.

3. Verwendung der Verbindungen I, ihrer Salze oder N-Oxide gemäß Anspruch 1 zur Herstellung von Mitteln gegen Schadpilze und tierische Schädlinge.

4. Verfahren zur Herstellung der Mittel gemäß Anspruch 2, **dadurch gekennzeichnet, daß** man eine Verbindung der allgemeinen Formel I mit mindestens einem Formulierungshilfsmittel verarbeitet.

5. Verwendung der Verbindungen I, ihrer Salze und N-Oxide gemäß Anspruch 1 oder der Mittel gemäß Anspruch 2, zur Bekämpfung von Schadpilzen und tierischen Schädlingen, wobei die Behandlung von Tieren ausgeschlossen bleibt.

6. Verfahren zur Bekämpfung von Schadpilzen und tierischen Schädlingen, **dadurch gekennzeichnet, daß** man die Schadpilze oder die tierischen Schädlinge, deren Lebensraum oder die von ihnen freizuhaltenden Pflanzen, Saatgüter, Flächen Materialien oder Räume mit einer wirksamen Menge einer Verbindung der Formel I oder eines ihrer Salze oder N-Oxide gemäß Anspruch 1 oder der Mittel gemäß Anspruch 2 behandelt, wobei die Behandlung von Tieren ausgeschlossen bleibt.

## Claims

1. A 2-(O-[pyrimidin-4-yl]methylenoxy)phenylacetic acid derivative of the formula I 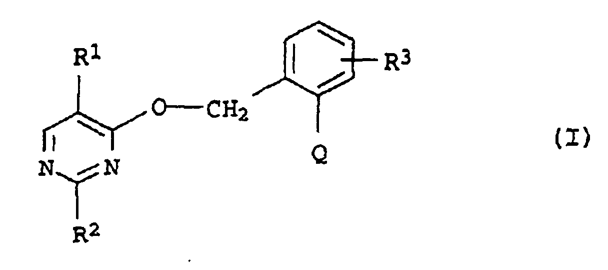 or a salt or N-oxide thereof where the radicals R¹ to R³ and Q have the following meanings:
R1 is halogen, C₁-C₂-alkyl or C₁-C₂-haloalkyl;
R2 is hydrogen; amino; hydroxyl; mercapto; halogen; C₁-C₈-alkyl, it being possible for the alkyl radicals to have attached to them a phenyl group which, in turn, can have attached to it one or, independently of one another, two of the following substituents: halogen, cyano, nitro, C₁-C₄-alkyl, C₁-C₄-haloalkyl and C₁-C₄-alkoxy; C₁-C₈-haloalkyl; C₁-C₈-alkoxy-C₁-C₄-alkyl; C₁-C₈-alkoxy; C₁-C₈-monoalkylamino; di-C₁-C₈-alkylamino; C₁-C₈-alkylthio; C₁-C₈-alkylsulfoxyl; C₁-C₈-alkylsulfonyl; C₃-C₈-cycloalkyl; tri-C₁-C₈-alkylsilyloxy or the following, unsubstituted or substituted in the aromatic ring: phenyl, phenoxy, phenoxymethyl, benzyloxy or hetaryl,
where R² is different from R¹;
R3 is hydrogen; cyano; halogen; C₁-C₄-alkyl; C₁-C₄-haloalkyl or C₁-C₄-alkoxy and
Q is C(=CHCH₃)-COOCH₃ or
C (=CHOCH₃)-COOCH₃.

2. A composition which is suitable for controlling harmful fungi and animal pests, comprising an effective amount of a compound of the formula I or of a salt or N-oxide thereof as claimed in claim 1 and at least one formulation auxiliary.

3. The use of a compound I or a salt or N-oxide thereof as claimed in claim 1 for the preparation of compositions against harmful fungi and animal pests.

4. A process for the preparation of a composition as claimed in claim 2, which comprises processing a compound of the formula I with at least one formulation auxiliary.

5. The use of a compound I or a salt or N-oxide thereof as claimed in claim 1 or of a composition as claimed in claim 2 for controlling harmful fungi and animal pests, the treatment of animals remaining exluded.

6. A method of controlling harmful fungi and animal pests, which comprises treating the harmful fungi or the animal pests, their environment, or the plants, seeds, areas, materials or spaces to be kept free from them, with an effective amount of a compound of the formula I or of a salt or N-oxide thereof as claimed in claim 1 or of a composition as claimed in claim 2, the treatment of animals remaining excluded.

## Revendications

1. Dérivés d'acide 2-(O-[pyrimidin-4-yl]méthylèneoxy)phénylacétique de formule générale I 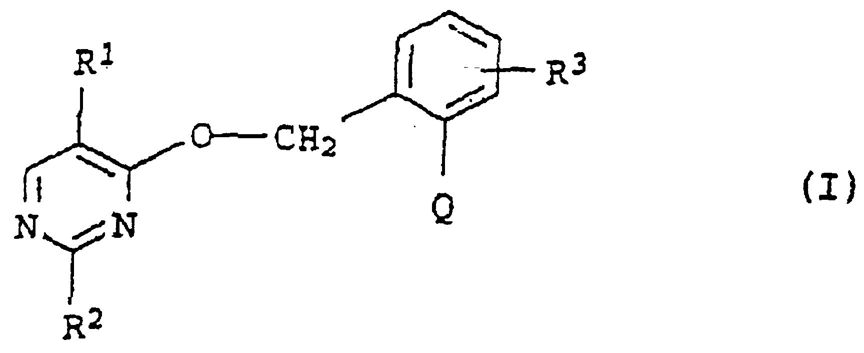 ainsi que leurs sels et N-oxydes, formule dans laquelle les radicaux R¹ à R³ et Q ont lés significations suivantes :
R¹ représente un atome d'halogène ou un groupe alkyle en C₁-C₂ ou halogénoalkyle en C₁-C₂ ;
R² représente un atome d'hydrogène ; le groupe amino, hydroxy, mercapto ; un atome d'halogène ; un radical alkyle en C₁-C₈, les radicaux alkyle pouvant porter un groupe phényle qui de son côté peut porter un ou, indépendamment l'un de l'autre, deux des substituants suivants : des atomes d'halogène, des groupes cyano, nitro, alkyle en C₁-C₄, halogénoalkyle en C₁-C₄ et alcoxy en C₁-C₄ ; un radical halogénoalkyle en C₁-C₈ ; alcoxy (C₁-C₈)-alkyle(C₁-C₄) ; alcoxy en C₁-C₈ ; monoalkyl(C₁-C₈)amino ; dialkyl(C₁-C₈)amino ; alkyl(C₁-C₈)thio ; alkyl(C₁-C₈)sulfoxyle ; alkyl(C₁-C₈)sulfonyle ; cycloalkyle en C₃-C₈ ; trialkyl(C₁-C₈)silyloxy ou, éventuellement substitué sur le noyau aromatique : un radical phényle, phénoxy, phénoxyméthyle, benzyloxy ou hétéroaryle ;
R² étant différent de R¹ ;
R³ représente un atome d'hydrogène ou d'halogène ou un groupe cyano, alkyle en C₁-C₄, halogénoalkyle en C₁-C₄ ou alcoxy en C₁-C₄ ; et
Q représente un groupe C(=CHCH₃)-COOCH₃ ou C(=CHOCH₃)-COOCH₃.

2. Composition appropriée à la lutte contre des champignons nuisibles et des ravageurs animaux, contenant une quantité efficace d'un composé de formule générale I ou de ses sels ou N-oxydes selon la revendication 1, et au moins un adjuvant de formulation.

3. Utilisation des composés I, de leurs sels ou N-oxydes selon la revendication 1, pour la préparation de compositions contre des champignons nuisibles et des ravageurs animaux.

4. Procédé pour la préparation des compositions selon la revendication 2, **caractérisé en ce qu'**on élabore un composé de formule générale I avec au moins un adjuvant de formulation.

5. Utilisation des composés I, de leurs sels et N-oxydes selon la revendication 1, ou des compositions selon la revendication 2, pour la lutte contre des champignons nuisibles et des ravageurs animaux, le traitement d'animaux étant exclu.

6. Procédé pour la lutte contre des champignons nuisibles et des ravageurs animaux, **caractérisé en ce qu'**on traite les champignons nuisibles ou les ravageurs animaux, leur habitat ou les plantes, semences, surfaces, matériaux ou espaces à libérer de ceux-ci, par une quantité efficace d'un composé de formule I ou d'un de ses sels ou N-oxydes selon la revendication 1 ou des compositions selon la revendication 2, le traitement d'animaux étant exclu.
